# EUROPEAN PATENT SPECIFICATION

(11) **EP 3 513 791 B1**
(45) Date of publication and mention of the grant of the patent: **28.07.2021**
(21) Application number: 17850873.5
(22) Date of filing: 12.09.2017
(51) Int. Cl.: A61K 31/4164, A61K 9/20, A61K 47/02, A61P 35/02

(54) **MEDICINAL DRUG, METHOD, USE, AND COMPOUND FOR PREVENTING OR CURING LEUKEMIA ASSOCIATED WITH MLL**
ARZNEIMITTEL, VERFAHREN, VERWENDUNG UND ZUSAMMENSETZUNG ZUR PRÄVENTION ODER BEHANDLUNG VON MLL-BEDINGTER LEUKÄMIE
MÉDICAMENT, MÉTHODE, UTILISATION ET COMPOSÉ POUR PRÉVENIR OU TRAITER LA LEUCÉMIE ASSOCIÉE AU MLL

(30) Priority: 13.09.2016 JP 2016178490; 01.12.2016 JP 2016233765
(43) Date of publication of application: 24.07.2019
(73) Proprietor: FUJIFILM Corporation, Tokyo 106-8620 (JP)
(72) Inventor: IWAMURA, Hiroyuki, Kanagawa 258-8577 (JP); SAITO, Motoki, Kanagawa 258-8577 (JP); GOYAMA, Susumu, Tokyo 113-8654 (JP); KITAMURA, Toshio, Tokyo 113-8654 (JP)
(74) Representative: Hoffmann Eitle
(86) International application number: PCT/JP2017/032828
(87) International publication number: WO 2018/051971

(56) References cited:
- WO-A1-2014/112530
- WO-A1-2015/105174
- WO-A1-2016/090271
- US-A1- 2014 275 568
- US-A1- 2015 057 324
- MOTOHIKO MURASE ET AL: "Lack of cross-resistance to FF-10501, an inhibitor of inosine-5'-monophosphate dehydrogenase, in azacitidine-resistant cell lines selected from SKM-1 and MOLM-13 leukemia cell lines", PHARMACOLOGY RESEARCH & PERSPECTIVES, vol. 4, no. 1, 28 January 2016 (2016-01-28), pages 1-13, XP055499503, GB ISSN: 2052-1707, DOI: 10.1002/prp2.206
- MATSUO Y ET AL: "Two acute monocytic leukemia (AML-M5a) cell lines (MOLM-13 and MOLM-14) with interclonal phenotypic heterogeneity showing MLL-AF9 fusion resulting from an occult chromosome insertion, ins (11;9) (q23;p22p23)", LEUKEMIA, NATURE PUBLISHING GROUP UK, LONDON, vol. 11, no. 9, 1 January 1997 (1997-01-01), pages 1469-1477, XP009502888, ISSN: 0887-6924, DOI: 10.1038/SJ.LEU.2400768
- MURASE, MOTOHIKO et al.: "Luck of cross-resistance to FF-10501, an inhibitor of inosine-5'-monophosphate dehydrogenase, in azacitidine-resistant cell lines selected from SKM-1 and MOLM-13 leukemia cell lines", Pharmacology Research and Perspectives, vol. 4, no. 1, 1 February 2016 (2016-02-01), pages 1-13, XP055499503, DOI: doi:10.1002/prp2.206
- MATSUO, YOSHINOBU et al.: "Two acute monocytic leukemia (AML-M5a) cell lines (MOLM-13 and MOLM-14) with interclonal phenotypic heterogeneity showing MLL-AF9 fusion resulting from an occult chromosome insertion , ins(11;9)(q23;p22p23", Leukemia, vol. 11, no. 9, 1997, pages 1469-1477, XP009502888, DOI: doi:10.1038/sj.leu.2400768
- KIMURA, KIYOJI et al.: "Zoketsuki Shuyo ni Taisuru SM-108(4-Carbamoylimidazolium-5-olate) no Phase II study", Japanese Journal of Cancer and Chemotherapy, vol. 16, no. 1, 1989, pages 123-130, XP008182427, ISSN: 0385-0684
- ZAMECNIKOVA, A.: "Acquisition of mixed lineage leukemia rearrangement in a chronic myeloid leukemia patient while on imatinib", Hematology Reports, vol. 3, 2011, pages 37-38, XP055499507, DOI: doi:10.4081/hr.2011.e13
- IIOKA FUTOSHI et al.: "Chronic myelomonocytic leukemia carrying t(11;19) (q23;p13.1); MLL-ELL fusion gene associated with ileocecal inflammatory disease", Tenri Medical Bulletin, vol. 15, no. 1, 2012, pages 53-61, XP055584178,

## Description

### BACKGROUND OF THE INVENTION

### 1. Field of the Invention

The present invention relates to a medicinal drug, and a compound; which are for preventing or curing leukemia associated with MLL.

### 2. Description of the Related Art

5-Hydroxy-1H-imidazole-4-carboxamide (hereinafter referred to as a compound A), or a salt thereof or a hydrate thereof has a strong anticancer action, and thus is a useful compound in medical treatment as an anticancer agent (Patent Documents 1-7). The compound A has been examined to be applied to diseases such as myelodysplastic syndrome (MDS), chronic myelogenous leukemia (CML), and acute myelogenous leukemia (AML) (Non-Patent Documents 1-2).

Leukemia having abnormality in a Mixed Lineage Leukemia (MLL) gene located in a 11q23 chromosomal region (11q23) is classified in a separate category from other types of leukemia by WHO classifications as leukemia associated with MLL, and has an extremely poor prognosis (Non-Patent Document 3). The mechanism of onset of this leukemia has not yet been sufficiently elucidated.

### Prior Art Documents

### Patent Documents

Patent Document 1: JP1978-032124A (JP-S53-032124A)
Patent Document 2: WO2009/035168A
Patent Document 3: WO2013/047758A
Patent Document 4: WO2014/112529A
Patent Document 5: WO2014/112530A
Patent Document 6: WO2014/112531A
Patent Document 7: JP2009-209129A

### Non-Patent Documents

Non-Patent Document 1: Cancer and chemotherapy, Vol. 16, No. 1, 123 to 130, 1989
Non-Patent Document 2: Cancer research, Vol. 42, pp. 1098 to 1102, 1982
Non-Patent Document 3: Genes & development, Vol. 23, pp. 877 to 889, 2009

### SUMMARY OF THE INVENTION

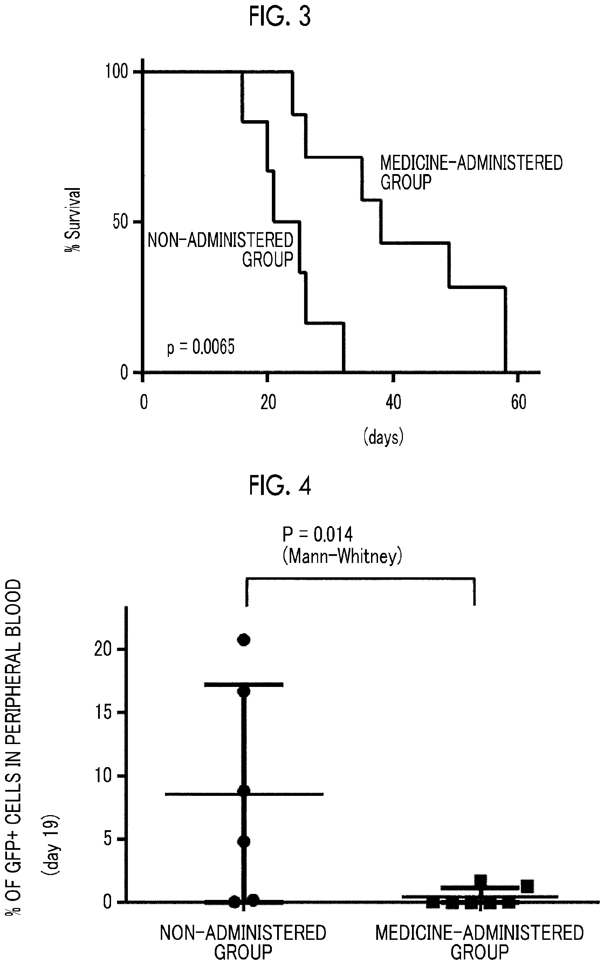

The leukemia associated with MLL has been known to be refractory, for which existing chemotherapy (cytarabine or doxorubicin) is not effective (Fig. 3 and Fig. 5 of Genes & development, Vol. 23, pp. 877 to 889, 2009), and a curing method thereof is desired. MLL-ENL mentioned in Genes & Development, Vol. 23, pp. 877 to 889, 2009 is the same MLL translocation-type fusion gene as MLL-AF9, and is perceived to have almost the same properties as those of MLL-AF9 in the field of basic research. An object of the present invention to provide a new medicinal drug, and a compound; which are for the refractory leukemia associated with MLL, for which existing chemotherapy is not effective.

Under such circumstances, the inventors of the present invention have conducted intensive research, and as a result, have found that the compound A, or a salt thereof or a hydrate thereof can achieve the above-described object, and therefore have completed the present invention.

The present invention provides the following aspects.
(1) A medicinal drug for preventing or curing leukemia associated with MLL, comprising 5-hydroxy-1H-imidazole-4-carboxamide, or a salt thereof or a hydrate thereof.
(2) The medicinal drug for preventing or curing leukemia associated with MLL, in which the leukemia associated with MLL is leukemia expressing a Mixed Lineage Leukemia fusion gene formed by fusion of a Mixed Lineage Leukemia gene with other genes. The medicinal drug for preventing or curing leukemia associated with MLL, in which the leukemia associated with MLL is acute myelogenous leukemia with (9;11) translocation or mixed phenotype acute leukemia with (v;11q23) translocation.

It is preferable that the medicinal drug for preventing or curing leukemia associated with MLL further contains an additive, and silicon dioxide is preferable as the additive. MLL.

The present invention is effective for preventing or curing leukemia associated with MLL.

### BRIEF DESCRIPTION OF THE DRAWINGS

Fig. 1 is a graph showing a survival period of a medicine-administered group and a non-administered group in a mouse acute myelogenous leukemia model into which an MLL fusion gene is introduced of Test Example 1.
Fig. 2 is a graph showing abundance of GFP⁺ cells in the peripheral blood on day 10 after MLL fusion gene leukemia cells of Test Example 1 have been injected to the mouse.
Fig. 3 is a graph showing a survival period of a medicine-administered group and a non-administered group in a mouse acute myelogenous leukemia model from which a p53 gene is deleted of Test Example 2.
Fig. 4 is a graph showing the abundance of GFP⁺ cells in the peripheral blood on day 19 after MLL fusion gene leukemia cells of Test Example 2 have been injected to the mouse.

### DESCRIPTION OF THE PREFERRED EMBODIMENTS

Hereinafter, the present invention will be described in detail.

The symbol "%" used in the present invention means mass percentage unless otherwise specified. A numerical value range indicated by using "to" in the present invention indicates a range including numerical values described before and after "to" as a minimum value and a maximum value. In a case where a plurality of substances corresponding to each component in the composition are present, an amount of each component in a composition in the present invention means a total amount of the plurality of substances present in the composition, unless otherwise specified.

The term "prevention" means inhibition of onset, reduction in risk of onset, delay of onset, and the like.

The term "curing" means improvement or suppression of progression of a target disease or condition.

The term "treatment" means preventing or curing various diseases.

The term "treatment agent" means a substance provided for the purpose of preventing or curing various diseases.

The present invention is a medicinal drug for preventing or curing leukemia associated with MLL, including 5-hydroxy-1H-imidazole-4-carboxamide (compound A), or a salt thereof or a hydrate thereof; a medicinal drug composition; or a treatment agent. In addition, the present invention is a medicinal drug for use in preventing or curing leukemia associated with MLL which contains the compound A, or a salt thereof or a hydrate thereof, a medicinal drug composition, or a treatment agent; or an anti-leukemia associated with MLL agent. A form thereof is preferably a tablet.

The leukemia associated with MLL means leukemia having abnormality in an MLL gene (Mixed Lineage Leukemia gene located in the 11q23 chromosomal region). The abnormality in the MLL gene refers to, for example, expression of an MLL fusion gene by fusion of the MLL gene with other different genes through chromosomal translocation. The chromosomal translocation that affects MLL, which is a proto-oncogene, occurs in both children and adults in high-grade human acute leukemia (refer to Sorensen et al., J Clin Invest. (1994), 93(1): pp. 429 to 437; Cox, et al., Am J Clin Pathol. (2004), 122(2): pp. 298 to 306).

In the present invention, the term "leukemia" means symptoms of leukemia and coexisting symptoms of leukemia.

In the present invention, the "leukemia" is a general term for diseases in which malignant hematopoietic cells proliferate indefinitely and thus are seen in the blood. In leukemia, leukemia in which a tumor cell loses differentiation ability is called acute leukemia, and leukemia in which a tumor cell maintains differentiation ability is called chronic leukemia. In addition, leukemia is classified into myelogenous leukemia in a case where a cell from which a tumor originated is a myelogenous cell, and lymphoid leukemia in a case where a cell is a lymphocyte. Based on the above description, leukemia is roughly classified into four types of leukemia of acute myelogenous leukemia (AML), chronic myelogenous leukemia (CML), acute lymphoid leukemia (ALL), and chronic lymphoid leukemia (CLL). Acute promyelocytic leukemia (APL), acute undifferentiated leukaemia (AUL), acute myelomonocytic leukemia (AMMoL), and juvenile chronic myelogenous leukemia (JCML) are also included in the classification. Furthermore, examples of the types of leukemia include acute monocytic leukemia (AMoL), chronic monocytic leukemia (CMoL), erythroleukemia, eosinophilic leukemia, basophilic leukemia, megakaryoblastic leukemia, plasmacytoid leukemia, chondroma, chronic neutrophilic leukemia, adult T cell leukemia, lymphosarcoma cell leukemia, hairy cell leukemia, and prolymphocytic leukemia. In the present invention, the types of leukemia are not particularly limited, and examples of the leukemia associated with MLL include leukemia expressing an MLL (Mixed Lineage Leukemia) fusion gene, which is refractory acute leukemia.

In MLL leukemia, the MLL gene fuses with other genes through specific chromosomal translocation seen in AML and ALL, and there are 40 or more translocation partners including AF4 and AF9, and p300/CBP. In addition, in a case of AML, the translocation is seen in FAB classification M4/M5 (monocytic leukemia). Furthermore, an increase in the expression of the HOX gene group has been known.

In regard to the leukemia associated with MLL, WHO classifications include acute myelogenous leukemia with (9;11) translocation or mixed phenotype acute leukemia with (v;11q23) translocation. Examples of the leukemia having abnormality in the MLL gene also include pediatric leukemia and acute lymphoid leukemia (ALL).

The compound A in the present invention indicates any one selected from the group consisting of the compound A, a salt of the compound A, a hydrate of the compound A, and a hydrate of the salt of the compound A in a case of referring to "compound A, or a salt thereof or a hydrate thereof' unless otherwise specified; and contains at least one selected from the group consisting of the compound A, the salt of the compound A, the hydrate of the compound A, and the hydrate of the salt of the compound A in a case where the medicinal drug contains "compound A, or a salt thereof or a hydrate thereof' unless otherwise specified.

The compound A, or the salt thereof or the hydrate thereof used in the present invention can be produced by, for example, a method described in Production Example 1 to be described later.

A content of compound A, or the salt thereof or the hydrate thereof can be from 0.3% to 95% with respect to a mass of the tablet, and is preferably from 20% to 90% and more preferably from 40% to 85%.

The medicinal drug, medicinal drug composition, or treatment agent of the embodiment of the present invention may further contain an additive.

The additive is not particularly limited, and examples thereof include silicon dioxide, a disintegrating agent, a binder, a lubricant, an excipient, a corrigent, a coloring agent, a flavoring agent, acid, a surfactant, and a plasticizer, of which silicon dioxide, a disintegrating agent, a binder, a lubricant, and an excipient are preferable. These additives may be used alone or in combination of two or more kinds thereof unless particularly specified. A formulation amount is not particularly limited, and the additive may be appropriately formulated depending on the purpose such that effects of the additive are sufficiently expressed.

Silicon dioxide can be formulated within a granulated powder and/or outside the granulated powder.

A content of silicon dioxide can be from 0.1% to 20% with respect to the mass of the tablet, and is preferably from 0.5% to 15% and more preferably from 1% to 5%.

Silicon dioxide is not particularly limited, and examples thereof include silica gel, silicic anhydride, colloidal silicon dioxide, light anhydrous silicic acid, and hydrous silicon dioxide, of which light anhydrous silicic acid and hydrous silicon dioxide are preferable.

Examples of the disintegrating agent include, but are not particularly limited to, cellulose derivatives such as carmellose calcium, carmellose, low substituted hydroxypropyl cellulose, and croscarmellose sodium; starch derivatives such as carboxymethyl starch sodium and partially pregelatinized starch; and polyvinylpyrrolidone derivatives such as crospovidone, of which carmellose calcium, low substituted hydroxypropyl cellulose, and partially pregelatinized starch are preferable, and carmellose calcium is more preferable.

The disintegrating agent can be formulated within a granulated powder and/or outside the granulated powder.

A content of the disintegrating agent can be from 1% to 20% of the mass of the tablet, and preferably from 3% to 15% and more preferably from 5% to 10%.

Examples of the binder include, but are not particularly limited to, hydroxypropyl cellulose, polyvinyl alcohol, povidone, hypromellose, carmellose sodium, methyl cellulose, gum arabic, and dextrin, of which hydroxypropyl cellulose and polyvinyl alcohol are preferable.

A content of the binder can be from 1% to 20% of the mass of the tablet, and is preferably from 2.5% to 10%.

Examples of the lubricant include, but are not particularly limited to, magnesium stearate, calcium stearate, stearic acid, sodium stearyl fumarate, talc, and sucrose fatty acid esters, of which magnesium stearate and sodium stearyl fumarate are preferable and magnesium stearate is more preferable.

A content of the lubricant can be from 0.1% to 5% with respect to the mass of the tablet, and is preferably from 0.2% to 5% and more preferably from 0.5% to 3%.

Examples of the excipient include, but are not particularly limited to, sugar alcohols such as erythritol, mannitol, xylitol, and sorbitol; sugars such as sucrose, powdered sugar, lactose, and glucose; cyclodextrins such as α-cyclodextrin, β-cyclodextrin, γ-cyclodextrin, hydroxypropyl-β-cyclodextrin, and sulfobutyl ether-β-cyclodextrin sodium; celluloses such as crystalline cellulose and microcrystalline cellulose; and starches such as corn starch, potato starch, and partially pregelatinized starch.

The corrigent is not particularly limited, and examples thereof include aspartame, saccharin, stevia, thaumatin, and acesulfame potassium.

The coloring agent is not particularly limited, and examples thereof include titanium dioxide, ferric oxide, yellow ferric oxide, black iron oxide, food red No. 102, food yellow No. 4, and food yellow No. 5.

Examples of the flavoring agent include, but are not particularly limited to, essential oils such as orange oil, lemon oil, peppermint oil, and pine oil; essences such as orange essence and peppermint essence; flavors such as cherry flavor, vanilla flavor, and fruit flavor; powdered perfumes such as apple micron, banana micron, peach micron, strawberry micron, and orange micron; vanillin; and ethyl vanillin.

The acid is not particularly limited, and examples thereof include hydroxycarboxylic acid, and citric acid, tartaric acid, and malic acid are preferable.

The surfactant is not particularly limited, and examples thereof include sodium lauryl sulfate, dioctyl sodium sulfosuccinate, polysorbates, and polyoxyethylene hydrogenated castor oil.

The plasticizer is not particularly limited, and examples thereof include triethyl citrate, macrogol, triacetin, and propylene glycol.

A surface of the tablet may be film-coated with a coating agent as necessary.

The coating agent is not particularly limited, and examples thereof include hypromellose, aminoalkyl methacrylate copolymer E, aminoalkyl methacrylate copolymer RS, ethyl cellulose, cellulose acetate phthalate, hypromellose phthalate ester, methacrylic acid copolymer L, methacrylic acid copolymer LD and methacrylic acid copolymer S, polyvinyl alcohol, hydroxypropylmethyl cellulose acetate succinate, polyvinyl alcohol-acrylic acid-methyl methacrylate copolymer, and polyvinyl alcohol-polyethylene glycol-graft copolymer, of which hypromellose and polyvinyl alcohol are preferable and hypromellose is more preferable.

Examples of the salt of the compound A according to the present invention include a salt in a commonly known basic group or acidic group.

Examples of the salt of the basic group include salts with mineral acid such as hydrochloric acid, hydrogen bromide, phosphoric acid, and sulfuric acid; salts with organic carboxylic acid such as tartaric acid, formic acid, acetic acid, fumaric acid, maleic acid, citric acid, trichloroacetic acid, and trifluoroacetic acid; salts with sulfonic acid such as methanesulfonic acid, benzenesulfonic acid, p-toluenesulfonic acid, mesitylenesulfonic acid, and naphthalenesulfonic acid; and the like.

Examples of the salt of acidic groups include salts with alkali metals such as sodium and potassium; salts with alkaline earth metals such as calcium and magnesium; ammonium salts; salts with nitrogen-containing organic bases such as trimethylamine, triethylamine, tributylamine, trometamol, pyridine, N,N-dimethylaniline, N-methylpiperidine, N-methylmorpholine, diethylamine, dicyclohexylamine, procaine, dibenzylamine, N-benzyl-β-phenethylamine, and N,N'-dibenzylethylenediamine; and the like.

Furthermore, among the above-described salts, preferable examples of salts of the compound A include pharmacologically acceptable salts.

Examples of the compound A, or the hydrate of the salt thereof according to the present invention include a hydrate of a compound A produced by a method described in JP1983-024569A (JP-S58-024569A), a hydrate of a compound A produced by a method described in WO2009/035168A, or a hydrate of a compound A produced by a method described in Production Example 1 to be described later, of which the hydrate of the compound A produced by the method described in Production Example 1 is preferable.

In a case of administering the medicinal drug of the embodiment of the present invention, an administration method, a dose, and administration frequency can be appropriately selected depending on the age, weight, and symptoms of a patient. The administration method may be any of oral administration or parenteral administration (for example, injection, infusion, administration to a rectal region, and the like). As the dose and the administration frequency, an amount capable of exerting the drug efficacy may be administered once or in divided doses per day. For adults, 10 to 5000 mg, preferably 200 to 2500 mg of the compound A per day can be administered once or in divided doses.

In a case where the medicinal drug of the embodiment of the present invention is a tablet, examples of a method for producing a tablet include a method in which a granulated powder is produced by a dry-type or wet-type granulation method, and as necessary, an excipient, a disintegrating agent, and/or a lubricant, and the like are further added thereto to form a mixed powder for tableting, and tableting is performed.

As a method for producing the granulated powder, a wet-type granulation method is preferable.

The wet-type granulation method is not particularly limited, and examples thereof include a fluidized bed granulation method, a centrifugal rolling granulation method, a mixing and stirring granulation method, a high speed mixing and stirring granulation method, a tumbling granulation method, a wet-type crushing granulation method, and an extrusion granulation method, of which a fluidized bed granulation method, a centrifugal rolling granulation method, a mixing and stirring granulation method, a high speed mixing and stirring granulation method, a tumbling granulation method, and a wet-type crushing granulation method are preferable, and a fluidized bed granulation method is particularly preferable.

In the case where the medicinal drug of the embodiment of the present invention is the tablet, a circular tablet is preferable as the tablet. The circular tablet may have a diameter of 5 to 9 mm and a thickness of 2 to 5 mm, and preferably has a diameter of 7 to 9 mm and a thickness of 3 to 5 mm.

In the case where the tablet is a circular tablet having a diameter of 8.5 mm, hardness thereof is preferably from 30 to 150 N, and more preferably from 50 to 130 N.

The present invention is a method for using 5-hydroxy-1H-imidazole-4-carboxamide, or a salt thereof or a hydrate thereof in treatment of leukemia associated with MLL, the method including a step of administering an effective therapeutic dose to a subject (mammals including humans) in need of such treatment. The present invention is a method for treating leukemia associated with MLL, the method including a step of administering an effective therapeutic dose of 5-hydroxy-1H-imidazole-4-carboxamide, or a salt thereof or a hydrate thereof to a subject (mammals including humans) in need of such treatment. Disclosed herein is the use of 5-hydroxy-1H-imidazole-4-carboxamide, or a salt thereof or a hydrate thereof for producing a medicinal drug for preventing or curing leukemia associated with MLL.

The present invention is 5-Hydroxy-1H-imidazole-4-carboxamide, or a salt thereof or a hydrate thereof, for use in preventing or curing leukemia associated with MLL.

### Examples

Next, the present invention will be described with reference to production examples, examples, and test examples, but the present invention is not limited thereto.

A 3/4 hydrate of the compound A produced according to the method described in Production Example 1 was used as the hydrate of the compound A.

### Production Example 1

(1) 30 g of 2-aminomalonamide (Tateyama Kasei Co., Ltd.) and 115 mg of oxalic acid were added to 600 mL of 2-propanol under a nitrogen atmosphere, and after heating to 82°C, 106 mL of triethyl orthoformate (NIPPOH CHEMICALS CO., LTD., purity: 99.5%) was added dropwise over 10 minutes. Next, the reaction mixture was stirred at 84°C for 7 hours 30 minutes. After cooling to 57°C, 30 mL of water and 24 mL of concentrated hydrochloric acid were successively added to the reaction mixture. The reaction mixture was cooled to 5°C, the crystals were collected by filtration and washed with 120 mL of acetone, and therefore 49 g of 5-hydroxy-1H-imidazole-4-carboxamide hydrochloride dihydrate of pale yellow crystals was obtained.
(2) Under a nitrogen atmosphere, 20.0 g of 5-hydroxy-1H-imidazole-4-carboxamide hydrochloride dihydrate was added to 240 mL of 0.45 mol/L hydrochloric acid, and dissolved by heating at 50°C. To this solution, a solution of 14.3 g of sodium formate in 40 mL of water was added dropwise over 33 minutes. The reaction mixture was cooled to 5°C, the crystals were collected by filtration, washed with a mixed solution of 20 mL of acetone and 40 mL of water, and then washed with 60 mL of acetone, and therefore 12.8 g of 5-hydroxy-1H-imidazole-4-carboxamide-3/4 hydrate was obtained as the pale yellow crystals.

Hereinafter, Test examples 1 and 2 will be described. Test Example 1 and 2 were carried out with reference to J Clin Invest., 2013 Sep 3, 123(9): 3876 to 3888; and Blood, 1 SEPTEMBER 2011, Volume 118, 2541 to 2550.

### Test Example 1

A mouse acute myelogenous leukemia (AML) model into which the MLL fusion gene (MLL-AF9) inducing cancer was introduced, was prepared. Specifically, bone marrow cells were collected from a C57BL/6-Ly5.1 mouse (Sankyo Labo Service Corporation, INC., hereinafter the same applies), and hematopoietic stem precursor cells were isolated using CD117 MicroBeads. MLL-AF9 marked with GFP (green fluorescent protein) was introduced into these cells using a virus. This MLL-AF9-introduced cells were transplanted to the same strain (C57BL/6) mouse (Japan SLC, Inc., hereinafter the same applies), and GFP-positive leukemia cells were collected from the mouse spleens that developed leukemia. These cells were subjected to second, third, and fourth transplantations, and after the fourth transplantation, MLL fusion gene (MLL-AF9) leukemia cells causing all the mice to develop leukemia in about 2 weeks were collected.

These MLL fusion gene (MLL-AF9) leukemia cells 1 × 10⁶/mouse were injected into the C57BL/6 mouse from the tail vein. On day 4 after the day of injection, 0.5% methylcellulose suspension of 5-hydroxy-1H-imidazole-4-carboxamide-3/4 hydrate (medicine) was administered orally to the mouse once per day at 160 mg/kg (in terms of free base) on Monday (day 4), Wednesday (day 6), and Friday (day 8), and this administration was continued during the survival period of the mouse. As a result, in a medicine-administered group, a significant extension of the survival period was observed as compared with a non-administered group.

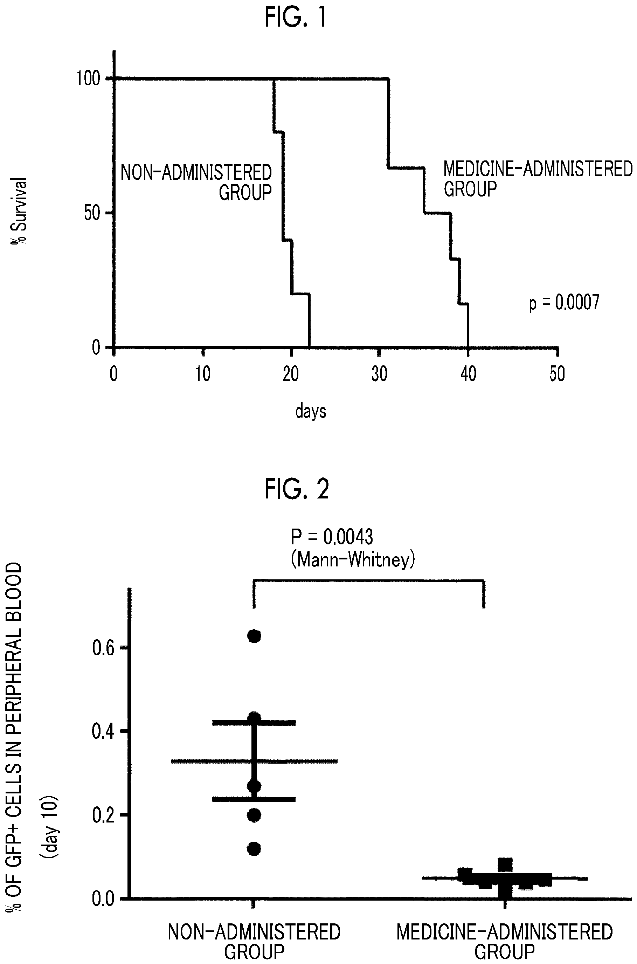

Fig. 1 is a graph showing the survival period of the medicine-administered group and the non-administered group in the mouse acute myelogenous leukemia model into which the MLL fusion genes were introduced of Test Example 1. Fig. 2 is a graph showing abundance of GFP+ cells in the peripheral blood on day 10 after the MLL fusion gene leukemia cells had been injected to the mouse of Test Example 1.

In other words, as shown in Fig. 1, it could be confirmed that all mice died in about 20 days in the non-administered group (control), whereas the mice were confirmed to survive for 30 days or longer in the medicine-administered group, and survived for up to 40 days. A log-rank test was performed regarding these results, and it was confirmed that the difference between the medicine-administered group and the non-administered group was statistically significant.

As also shown in Fig. 2, it could be confirmed that GFP+ cells proliferated in the non-administered group (Control), whereas proliferation of GFP+ cells was suppressed in the medicine-administered group. Based in the above results, it was verified that proliferation of cancer cells was suppressed in the medicine-administered group. A Mann-Whitney's U test was performed regarding these results, and it was confirmed that the difference between the medicine-administered group and the non-administered group was statistically significant.

### Test Example 2

In the case where the survival period was extended depending on the function of a p53 gene, there is a concern that clinical usefulness may be limited. For this reason, the MLL fusion gene (MLL-AF9) leukemia cells were prepared in the same manner as in Test Example 1 by using mice in which the p53 gene was deleted. The survival periods of a 5-hydroxy-1H-imidazole-4-carboxamide-3/4 hydrate (medicine)-administered group and a non-administered group were confirmed in the same manner as in Test Example 1. As a result, it could be confirmed that the extension of the survival period according to the present invention does not depend on the p53 gene. In other words, the usefulness for p53-deleted leukemia cells was found.

Fig. 3 is a graph showing the survival period of the medicine-administered group and the non-administered group in the mouse acute myelogenous leukemia model from which the p53 gene was deleted of Test Example 2. Fig. 4 is a graph showing the abundance of GFP+ cells in the peripheral blood on day 19 after MLL fusion gene leukemia cells had been injected to the mouse of Test Example 2.

In other words, as shown in Fig. 3, it could be confirmed that the mice in the non-administered group (Control) died in less than 20 days, whereas the mice in the medicine-administered group survived for 20 days or longer, or even for up to 40 days or longer.

As also shown in Fig. 4, it could be confirmed that GFP+ cells proliferated in the non-administered group (Control), whereas proliferation of GFP+ cells was suppressed in the medicine-administered group. Based in the above results, it was verified that proliferation of cancer cells was suppressed in the medicine-administered group.

The present invention is useful as a therapeutic agent for refractory leukemia associated with MLL, for which existing chemotherapy is not effective.

## Claims

1. 5-Hydroxy-1H-imidazole-4-carboxamide, or a salt thereof or a hydrate thereof for use in preventing or curing leukemia associated with MLL.

2. The 5-Hydroxy-1H-imidazole-4-carboxamide, or a salt thereof or a hydrate thereof for use according to claim 1, wherein the leukemia associated with MLL is leukemia expressing a Mixed Lineage Leukemia fusion gene formed by fusion of a Mixed Lineage Leukemia gene with other genes.

3. The 5-Hydroxy-1H-imidazole-4-carboxamide, or a salt thereof or a hydrate thereof for use according to claim 1 or 2, wherein the leukemia associated with MLL is acute myelogenous leukemia with (9;11) translocation or mixed phenotype acute leukemia with (v;11q23) translocation.

## Patentansprüche

1. 5-Hydroxy-1H-imidazol-4-carboxamid oder ein Salz davon oder ein Hydrat davon zur Verwendung bei der Vorbeugung oder Heilung von mit MLL assoziierter Leukämie.

2. 5-Hydroxy-1H-imidazol-4-carboxamid oder ein Salz davon oder ein Hydrat davon zur Verwendung gemäß Anspruch 1, worin die mit MLL assoziierte Leukämie eine Leukämie ist, die ein Mixed-Lineage-Leukämie-Fusionsgen exprimiert, gebildet durch Fusion eines Mixed-Lineage-Leukämie-Gens mit anderen Genen.

3. 5-Hydroxy-1H-imidazol-4-carboxamid oder ein Salz davon oder ein Hydrat davon zur Verwendung gemäß Anspruch 1 oder 2, worin die mit MLL assoziierte Leukämie akute myeloische Leukämie mit (9;11)-Translokation oder akute Leukämie mit gemischtem Phänotyp mit (v;11q23)-Translokation ist.

## Revendications

1. 5-hydroxy-1H-imidazole-4-carboxamide, ou un sel de celui-ci ou un hydrate de celui-ci à utiliser dans la prévention ou le traitement de la leucémie associée à la MLL.

2. 5-hydroxy-1H-imidazole-4-carboxamide, ou un sel de celui-ci ou un hydrate de celui-ci à utiliser selon la revendication 1, dans lequel la leucémie associée à la MLL est une leucémie exprimant un gène de fusion de leucémie de lignée mixte formé par la fusion d'un gène de leucémie de lignée mixte avec d'autres gènes.

3. 5-hydroxy-1H-imidazole-4-carboxamide, ou un sel de celui-ci ou un hydrate de celui-ci à utiliser selon les revendications 1 ou 2, dans lequel la leucémie associée à la MLL est une leucémie myéloïde aiguë avec une translocation (9 ; 11) ou une leucémie aiguë de phénotype mixte avec une translocation (v ; 11q23).
